(19) Europäisches Patentamt
European Patent Office
Office européen des brevets

(11) Numéro de publication : **0 635 276 A1**

(12) # DEMANDE DE BREVET EUROPEEN

(21) Numéro de dépôt : **94401631.0**

(22) Date de dépôt : **13.07.94**

(51) Int. Cl.[6] : **A61L 25/00,** A61L 27/00, A61L 31/00

(30) Priorité : **21.07.93 FR 9308964**

(43) Date de publication de la demande : **25.01.95 Bulletin 95/04**

(84) Etats contractants désignés : **AT BE CH DE DK ES FR GB GR IE IT LI LU NL PT SE**

(71) Demandeur : **IMEDEX S.A.**
**Zone Industrielle des Troques**
**F-69630 Chaponost (FR)**

(72) Inventeur : **Constancis, Alain**
**17 rue Saint Nestor**
**I-69008 Lyon (FR)**
Inventeur : **Soula, Gérard**
**33, rue Nungesser**
**F-69330 Meyzieu (FR)**
Inventeur : **Tayot, Jean-Louis**
**1 rue des Greffières**
**F-69890 La Tour de Salvagny (FR)**
Inventeur : **Tiollier, Jerôme**
**41 Quai Jayr**
**F-69009 Lyon (FR)**

(74) Mandataire : **Bernasconi, Jean et al**
**Cabinet Bernasconi et Vigier**
**13 boulevard des Batignolles**
**F-75008 Paris (FR)**

(54) **Nouvelles compositions adhesives à usages chirurgical.**

(57) L'invention concerne une composition adhésive, biocompatible, biodégradable et non toxique, à usage chirurgical, notamment pour la liaison de tissus de formule générale suivante :

(I) 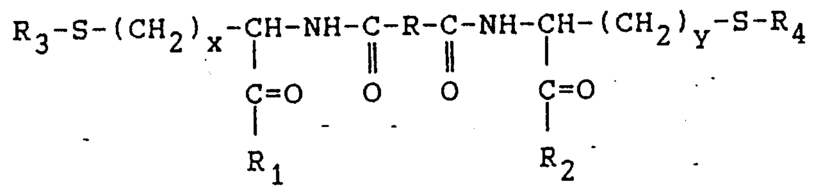

dans laquelle :
— R est une chaîne hydrocarbonée, comportant de 1 à 50 atomes de carbone,
— $R_1$ et $R_2$ sont identiques ou différents et sont choisis parmi les groupements suivants :
$-O-R_5$ ;

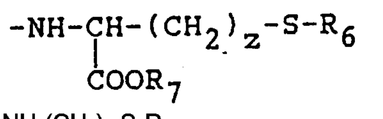 ;

$-NH-(CH_2)_y-S-R_6$
— $R_3$, $R_4$, $R_5$, $R_6$, et $R_7$, représentent, indépendamment, l'hydrogène ou un groupe aliphatique et/ou alicyclique et/ou aromatique,
$-CH_3$ ; $-CH_2-CH_3$ ; $-CH_2\phi$ ;

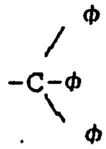

EP 0 635 276 A1

La présente invention concerne de nouvelles compositions adhésives comprenant des produits résultant, par exemple, de la condensation d'un diacide carboxylique avec un acide aminé soufré ou l'un de ses dérivés. Ces produits comportent des fonctions thiols SH réactives pouvant s'oxyder pour former des ponts disulfures, conduisant à des polymères, réticulés ou non.

On connaît déjà des polymères et des oligomères synthétiques biodégradables, qui sont constitués, très souvent, d'enchaînements simples et hydrolysables (esters ou amides) de composés propres à se dégrader en formant des métabolites.

Ainsi, la demande de brevet EP 0 332 530 décrit des polymères hydrophiles, de degré de polymérisation inférieur à 1 000, de préférence compris entre 20 et 300, et constitués par des polyamides résultant de la condensation de l'acide citrique avec des diamines, tels que la lysine, la cystamine, la cystine.

La synthèse de ces polyamides présente des difficultés réelles liées à la protection puis à la déprotection de l'acide citrique.

Ces polyamides biodégradables sont utilisables pour la préparation de vecteurs de médicaments, de sutures, de ligatures ou de prothèses, ou bien encore d'adhésifs chirurgicaux.

Si pour certaines applications, l'utilisation de polymères de masses relativement élevées, du type de ceux décrits dans la demande de brevet EP 0 332 530, est intéressante, pour d'autres usages, l'emploi de monomères ou d'oligomères porteurs de fonctions réactives ou polymérisables (prépolymères) est préférable. C'est le cas, en particulier, en chirurgie réparatrice (comblement osseux, ciments chirurgicaux, adhésifs biologiques...) ou en chirurgie dentaire (ciments dentaires...). Dans ces applications, il est intéressant que le monomère ou prépolymère puisse diffuser très facilement dans le tissu à réparer et pénétrer ainsi dans tous les espaces interstitiels. La polymérisation peut ensuite intervenir "in situ" et donner naissance à un enchevêtrement de chaînes polymères ayant les propriétés de comblement, de cohésion ou d'adhésion désirées.

Dans cet état de la technique, l'un des objectifs essentiels de l'invention est de fournir des produits synthétiques organiques, chirurgicaux adhésifs à base de produits atoxiques, biocompatibles et biodégradables.

Un autre objectif essentiel de l'invention est de fournir de tels produits comprenant des produits synthétiques organiques qui se trouvent sous forme de prépolymères et/ou de monomères, aptes à diffuser aisément dans les tissus biologiques et à polymériser in situ, voire in vivo, pour assurer, de façon satisfaisante, les fonctions d'adhésion.

Ces objectifs et d'autres sont atteints par la présente invention qui concerne, en premier lieu, une composition adhésive, biocompatible, biodégradable et non toxique, à usage chirurgical interne ou externe, qui comprend un produit organique contenant au moins deux fonctions thiols ou dérivés et des fonctions carboxyliques, protégées ou non, et/ou des fonctions carbonylées, de formule générale suivante :

(I)

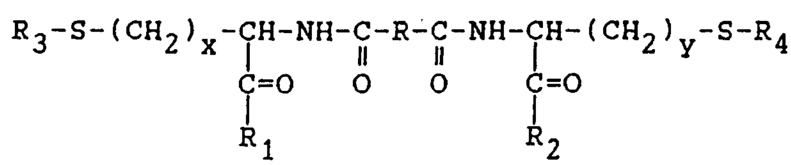

dans laquelle :

- R est une chaîne hydrocarbonée, de préférence alkylée, comportant de 1 à 50 atomes de carbone et, plus préférentiellement encore, aliphatique ayant de 1 à 10 atomes de carbone,
- $R_1$ et $R_2$ sont identiques ou différents et sont choisis parmi les groupements suivants :
  - $-O-R_5$ ;

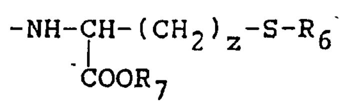 ;

$-NH-(CH_2)_y-S-R_5$

- $R_3$ ; $R_4$ ; $R_5$ ; $R_5$ et $R_7$ représentent, indépendamment, l'hydrogène ou un groupe aliphatique et/ou alicyclique et/ou aromatique, de préférence un groupe alkyle inférieur et/ou un groupe aromatique et, plus préférentiellement encore, l'un des groupements suivants :

$$-CH_3 \; ; \; -CH_2-CH_3 \; ; \; -CH_2-\phi \; ; \; -C(\phi)_3$$

- x, y, z = 1 ou 2.

Pour des raisons de simplification, on désigne les cycles aromatiques par la lettre grecque $\phi$ dans tout le présent exposé.

Au sens de la présente invention, le terme "alkyle inférieur" désigne des radicaux comprenant de 1 à 6 atomes de carbone.

Les composés biologiques répondant à cette formule ont, avantageusement, une masse moléculaire relativement faible (inférieure à 2 000) et peuvent donc diffuser aisément à travers les réseaux de protéines (collagène, élastine...) ou glycoprotéines constituant les tissus. C'est une propriété qu'il est intéressant d'exploiter dans le domaine des adhésifs.

Une première sous-famille des produits utilisés dans le cadre de l'invention comprend ceux dans lesquels les radicaux $R_1$ et $R_2$ représentent $OR_5$.

Plus précisément encore, dès lors que $R_3$ et $R_4$ correspondent à l'hydrogène, on est en présence d'un oligomère présentant, à chacune de ses deux extrémités, une fonction SH portée par un motif cystéine ou dérivé (oligomère "di SH").

Ces fonctions SH ont la faculté de pouvoir réagir entre elles, pour former des ponts disulfures et permettre l'obtention de longues chaînes. Cette propriété peut être exploitée pour préparer divers produits adhésifs tels que des fils, films ou des solutions visqueuses biodégradables.

La présence de fonctions carboxyliques sur ces composés di SH, permet d'envisager des interactions avec d'autres molécules (par exemple macromolécules naturelles). Ceci va dans le sens de l'amélioration des propriétés adhésives. En outre, ces fonctions carboxyliques amènent un caractère hydrophile et une capacité à fixer des principes actifs.

Une deuxième sous-famille typique des produits utilisés dans le cadre de l'invention regroupe les produits répondant à la formule générale sus-indiquée, dans laquelle le radical $R_1$ représente :

$$-NH-\underset{\displaystyle COOR_7}{\underset{|}{CH}}-(CH_2)_z-S-R_6$$

et $R_2$ représente $-O-R_5$ ou inversement.

Dès lors que $R_5$ et $R_6$ sont constitués par l'hydrogène, on peut qualifier ces composés d'oligomères "tri SH". Ces oligomères, dont les extrémités SH sont susceptibles de réagir pour former des ponts disulfures, laissent entrevoir des possibilités d'élaboration de réseaux multidirectionnels, qui ne peuvent qu'améliorer les propriétés mécaniques, les vertus d'adhésion et la résistance à la biodégradation des produits selon l'invention.

Une troisième sous-famille de produits organiques utilisés dans le cadre de l'invention est constituée par les produits dans lesquels les radicaux $R_1$ et $R_2$ sont constitués par le radical :

$$-NH-\underset{\displaystyle COOR_7}{\underset{|}{CH}}-(CH_2)_z-S-R_6$$

Avec $R_3$ $R_4$ et $R_6$ correspondant à l'hydrogène, on définit un oligomère tétra fonctionnel, comportant quatre motifs SH à ses extrémités (oligomère "tétra SH"). Cette multiplicité de points d'ancrage potentiels est avantageusement exploitable dans le domaine des biomatériaux. Cela s'inscrit dans le prolongement de ce qui a été indiqué ci-dessus pour les oligomères di et tri-fonctionnels.

Le motif cystélque mis en oeuvre peut être formé par la cystéine en elle-même : x, y, z = 1 ou par l'homocystéine : x, y, z = 2, qui peuvent être, éventuellement, issus de cystine ou d'homocystine.

La chaîne alkylée R, éventuellement substituée, définit le radical :

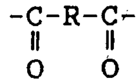

dans la formule (I), de telle sorte qu'il appartienne à la famille des restes d'acides poly, avantageusement di, carboxyliques, à l'exclusion de l'acide citrique, R étant, de préférence, sélectionné parmi les groupements suivants :

$-(CH_2)_p$ ;

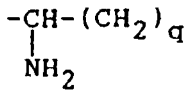 ; 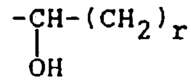

avec :

- $p \leqq 5$, de préférence, égal à 2 (acide succinique) ou à 3 (acide glutarique),
- $q \leqq 5$, de préférence, égal à 1 (acide aspartique) ou à 2 (acide glutamique),
- et enfin, $r \leqq 5$, de préférence, égal à 1 (acide malique).

R peut être, également, composé d'enchaînements polylactique et/ou polyglycolique et/ou polyaminoacide, de faible masse moléculaire.

Ces oligomères utilisés dans le cadre de l'invention sont porteurs de fonctions SH leur conférant des aptitudes à la polymérisation et/ou à la réticulation, éventuellement en présence d'un agent oxydant. Ils permettent donc d'obtenir, après oxydation, des polymères, réticulés ou non, utilisables comme biomatériaux et éventuellement dégradables en métabolites naturels, i. e.. qui interviennent dans les cycles biologiques des mammifères.

Par ailleurs, leur taille et leur structure sont telles qu'ils peuvent facilement migrer et pénétrer dans les tissus biologiques des mammifères.

Il s'ensuit que ces oligomères peuvent accéder sans difficulté jusqu'aux sites biologiques visés et polymériser "in situ" de manière à former un enchevêtrement et/ou un réseau de chaines polymères.

Ces oligomères trouvent donc leur utilisation en tant que constituants des compositions ou matériaux adhésifs selon l'invention.

De plus, la polymérisation de ces produits par oxydation des SH en ponts disulfures peut, également, être effectuée in vitro et permettre ainsi la formation de films ou d'objets moulables utilisables en tant que biomatériaux restant adhésifs.

La présente invention concerne, également, un biomatériau adhésif comprenant un ou des polymères susceptibles d'être obtenus à partir des oligomères, tels que décrits ci-dessus et qui répondent à la formule générale suivante :

II) 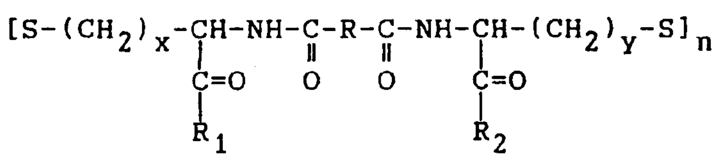

dans laquelle :

- $R_1$ et $R_2$ sont identiques ou différents et sont choisis parmi les groupements suivants :

  $-O-R_5$ ;

  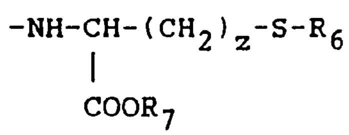 ;

  $-NH-CH_2-CH_z-S-R_6$ ;

  avec $R_5$, $R_5$, $R_7$ représentant, indépendamment, l'hydrogène ou un groupe aliphatique et/ou alicyclique et/ou aromatique, de préférence un groupe alkyle inférieur et/ou un groupe aromatique et, plus préférentiellement encore, l'un des groupements suivants :

$-CH_3$ ; $-CH_2CH_3$ ; $-CH_2-\phi$ ;

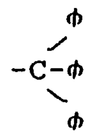 ;

- R est choisi de telle sorte que le radical :

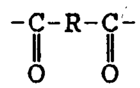

de la formule (I) soit un radical appartenant à la famille des acides poly, avantageusement di, carboxyliques, à l'exclusion de l'acide citrique, de préférence sélectionné parmi les groupements suivants :

$-(CH_2)_p-$ ;

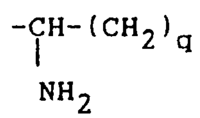 ; 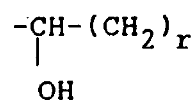

avec :

- $p \leqq 5$, de préférence, égal à 2 ou 3,
- $q \leqq 5$, de préférence, égal à 1 ou 2,
- et $r \leqq 5$, de préférence, égal à 1,
- n étant compris entre 1 et 100, de préférence entre 2 et 50 et, plus préférentiellement encore, entre 4 et 30,
- et x et y correspondant à 1 ou 2 comme précedémment.

R peut être, également, composé d'enchaînements polylactique et/ou polyglycolique et/ou polyaminoacide, de faible masse moléculaire.

Ces polymères sont des polysulfures dont l'unité récurrente résulte, de préférence, de l'association d'acide succinique et de cystéine.

Ces polymères peuvent servir de base à l'obtention d'autres produits adhésifs conformes à l'invention en constituant des réticulats (III). Cette réticulation s'effectue, par exemple, par amidation et/ou estérification, à l'aide d'au moins un agent de pontage, de préférence choisi parmi les produits suivants : cystine, lysine, cystamine et leurs dérivés, oses et leurs dérivés hydrogénés et autres polyols (glycérol).

L'invention concerne également , à titre de produits nouveaux, les biomatériaux adhésifs contenant les réticulats (III) réticulés par des ponts issus d'au moins un agent de pontage du type de celui évoqué ci-dessus.

Etant donné que tous les produits conformes à l'invention décrits ci-dessus peuvent s'intégrer dans une même chaîne de préparation, il est clair que la présente invention concerne aussi toute composition bioadhésive constituée par un mélange d'au moins deux des susdits produits, y compris par le mélange d'un polymère ou d'un réticulat avec un produit non solide tel que défini précédemment, ou par l'imprégnation d'un tel polymère ou réticulat à l'aide d'un tel produit non solide.

Les colles selon l'invention sont biocompatibles et se sont révélés être particulièrement appropriés pour entrer dans la composition de biomatériaux.

La présente invention a ainsi également pour objet tout biomatériau adhésif formé d'un mélange et/ou d'une association d'au moins l'un des oligomères (I) et/ou polymères (II), et/ou réticulats (III) et/ou des compositions décrits ci-avant avec des macromolécules biologiques, des polypeptides synthétiques ou naturels biodégradables, tels que :

- les polysaccharides ; e.g. amidon, cellulose, chitosane, dextrane, mucopolysaccharides tels que l'acide hyaluronique ou chondroïtine sulfate ;
- les protéines ; e.g. collagène, gélatine, albumine, globulines ;
- les polyaminoacides ;
- les polyesters (notamment lactiques et/ou glycoliques), les polyorthoesters, les polyanhydrides, les polyphosphazènes ;
- et les lipides et phospholipides.

Dans ces mélanges et/ou associations, ces macromolécules peuvent être engagées dans des liaisons physiques et/ou chimiques, avec les produits I, II, III et les compositions selon l'invention.

Ces biomatériaux adhésifs peuvent être des colles ou des matériaux collants sous toute forme physique, y compris solide.

Les compositions selon l'invention peuvent être utilisées, in vitro ou in vivo, pour la liaison de tissus biologiques entre eux ou d'un tissu biologique et d'un biomatériau implanté, y compris lorsque le tissu biologique est fortement hydraté.

Dans une première forme de réalisation, la composition se présente sous forme de solution liquide, par exemple en flacon ou en spray, ou sous une forme analogue à la forme liquide, par exemple sous forme de gel ou de particules de très petites dimensions. Dans cette forme de réalisation, la fonctionalité d'adhésion est assurée par un monomère polyfonctionel (multi-SH et multi-COOH) pouvant diffuser dans les tissus biologiques à encoller.

Pour assurer la prise de la colle ainsi réalisée, on amène un agent oxydant capable d'induire la polymérisation de ce monomère. Cet agent oxydant peut être par exemple, une solution d'iode, d'eau oxygénée, une enzyme oxydante (oxydase) ou même l'oxygène lui-même, sous forme pure ou sous forme atmosphérique.

La composition peut, par exemple, se présenter sous forme de kit comprenant, dans un récipient, la composition adhésive, et dans l'autre un agent oxydant.

Pour la liaison de tissus entre eux ou d'un tissu et d'un biomatériau implanté, consistant à appliquer l'une contre l'autre les deux surfaces à réunir, on applique ou on laisse diffuser, sur au moins l'une desdites surfaces, une composition et/ou un agent oxydant dans des conditions telles que l'agent oxydant entraîne une polymérisation de la composition au moment où lesdites surfaces sont appliquées l'une contre l'autre. Par exemple la composition peut être appliquée sur une surface et l'agent oxydant sur l'autre. Ou bien l'agent oxydant est appliqué d'abord seul sur l'une ou sur les deux surfaces puis ensuite la composition est appliquée entre les deux tissus.

On peut aussi mélanger la composition et l'agent oxydant au moment de la mise en place, ou peu avant ce moment, par exemple en utilisant une double seringue ou tout autre dispositif de mélange extemporané.

Quels que soient le mode et la séquence des applications sur les surfaces, le résultat doit être tel que l'agent oxydant et la composition se trouvent en contact intime au niveau des deux surfaces à réunir, la composition et/ou l'agent étant de préférence conçu sous une forme permettant une certaine diffusion depuis la surface vers l'intérieur du tissu.

Dans un autre mode de mise en oeuvre la composition se présente sous forme d'un matériau massif, dont la forme géométrique peut être très variable. Il peut s'agir par exemple d'un film ou d'un textile, d'une éponge, d'un patch ou toute autre forme. Dans cette forme de réalisation, dans laquelle la composition est sous forme de solide massif, le produit organique est avantageusement un polymère selon la formule (II) ayant des propriétés adhésives.

Les compositions selon l'invention peuvent être associées à un biomatériau, de préférence résorbable, pour former un biomatériau complexe présentant, superficiellement ou dans sa profondeur, une composition adhésive selon l'invention. La composition peut être présente dans ou sur le biomatériau soit en étant incorporée lors de la fabrication du biomatériau, soit par imprégnation, enduction ou tout autre procédé.

Le biomatériau, auquel est associé la composition, peut ne former qu'un vecteur biorésorbable, par exemple en collagène, pouvant avoir une forme physique quelconque, par exemple sous forme de suspension, microbilles, gel, film, éponge, patch, pour former, avec la composition, le biomatériau complexe destiné à être appliqué entre les tissus. Mais en variante ce biomatériau peut former une prothèse ou un autre élément plus durable, par exemple agent de comblement, associé à la composition selon l'invention permettant son collage à un ou plusieurs tissus. Ces biomatériaux complexes sont mis en place dans le tissu, ou entre les tissus, en présence d'un agent oxydant.

L'invention concerne également les biomatériaux précités, dépourvus de composition selon l'invention, ces biomatériaux présentant superficiellement ou dans leurs profondeurs, un agent oxydant destiné à réagir avec une composition selon l'invention. Dans ce cas le collage s'effectue en amenant une composition, par exemple liquide ou sous forme de gel, au contact du biomatériau ou de la surface tissulaire contre lequel le biomatériau est appliqué, de façon à provoquer la réaction entre l'agent oxydant et la composition.

Le biomatériau non adhésif auquel on associe la composition adhésive selon l'invention pour former un biomatériau adhésif complet, peut consister en, ou comporter un matériau biocompatible quelconque, et de préférence en collagène. Cependant, dans une variante intéressante, ce biomatériau peut être lui-même principalement ou entièrement constitué d'un polymère adhésif, ou non, selon la formule (II) ou d'un réticulat (III) auquel on associe, par mélange ou tout autre moyen, une composition ou matériau adhésif selon l'invention.

En effet le polymère selon la formle (II) peut être réalisé dans des conditions conduisant à une polymérisation ne laissant subsister que peu ou pas de fonctions adhésives.

L'obtention des produits adhésifs (I), (II) et (III) s'intègre dans un schéma réactionnel mis au point par la société française FLAMEL TECHNOLOGIES SA, et qui est le suivant : la première étape est la préparation de polymères dont, notamment, ceux répondant à la formule (II), qui donnent accès ensuite aux produits (I), eux-mêmes retransformables en polymères (II) ou en réticulats (III).

De préférence cette préparation consiste à effectuer :

a) une polycondensation entre :

- d'une part, un réactif de formule A :

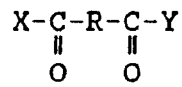

. avec X et Y, identiques ou différents et représentant un halogène, de préférence le chlore ou un radical - $OR_8$, dans lequel $R_8$ correspond à l'hydrogène ou à un radical alicyclique ou aliphatique, de préférence choisi parmi la liste de radicaux suivants :

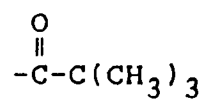 ; 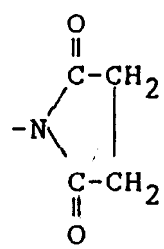 ; 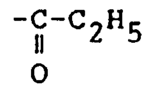

. et avec un radical R qui est une chaîne hydrocarbonée, de préférence alkylée, comportant de 1 à 50 atomes de carbone et, plus préférentiellement encore, aliphatique ayant de 1 à 10 atomes de carbone,

. et, d'autre part, un réactif de formule B :

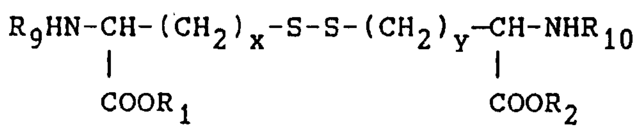

. avec $R_1$ et $R_2$ répondant à une définition identique à celle donnée précédemment,

. avec $R_9$ et $R_{10}$ identiques ou différents et choisis parmi les radicaux suivants : H, aliphatiques, de préférence alkyles, l'hydrogène étant encore celui qui est plus préférentiellement retenu,

. et avec x et y étant, classiquement, égal à 1 ou 2,

b) une réduction du polymère obtenu, ultérieurement transformé ou non.

En pratique, il est préférable que le composé de formule A soit sous forme d'un halogénure d'acide, par exemple d'un chlorure d'acide, et que le composé B de nature cystéique soit estérifié avec des radicaux alkyles $R_1$ et $R_2$ constitués, de préférence, par des radicaux méthyles.

Deux techniques de polycondensation sont envisageables pour obtenir des polymères dont ceux de formule (II) : polycondensation en solution ou polycondensation interfaciale.

Ces techniques seront vues en détail dans les exemples ci-après.

Une fois le polymère obtenu, il est avantageux de procéder à une hydrolyse des fonctions esters portées par ce polymère. Cette hydrolyse est réalisée dans l'eau, en milieu alcalin doux, afin de ménager les fonctions autres que les fonctions esters du polymère (saponification).

Suivant une première variante du procédé, le polymère, ayant subi ou non une hydrolyse de ses fonctions esters, est soumis à une réduction des ponts disulfures qu'il comporte, ce qui permet d'obtenir majoritairement des oligomères difonctionnels porteurs d'un motif SH à chacune de leurs extrémités.

Les techniques de réduction mises en oeuvre sont conventionnelles. Il peut s'agir, par exemple, de celles décrites dans METHODS IN ENZYMOLOGY, vol. 143, "Sulfur and sulfur amino-acids", W.B. JAKOBY, O.W. GRIFFITH, Academic Press Inc., Orlando, (1987).

Suivant une deuxième variante du procédé, on soumet le polymère partiellement ou totalement saponifié

à une réticulation. Ce polymère peut être le polycondensat tel quel ou réduit conformément à la première variante du procédé ce qui correspond aux oligomères difonctionnels di SH. La réticulation s'opère par l'intermédiaire d'au moins un agent de pontage et, de préférence, en présence d'un agent de couplage.

L'agent de pontage est, de préférence, un diol ou une diamine présentant au moins une liaison -S-S-, comme par exemple la cystine dialkyl ester (méthyle ou éthyle).

L'agent de couplage est, avantageusement, choisi dans la liste de composés suivants : éthyl diaminopropyl carbodiimide (EDC), carbonyldiimidazole (CDI).

On peut faire varier le taux de réticulation en jouant sur la quantité d'agent de pontage mis en oeuvre, par rapport au nombre de fonctions acides du polymère.

La concentration en agent de pontage est définie par le ratio suivant :

$$\frac{\text{nombre de fonctions } NH_2\text{, OH... de l'agent pontant}}{\text{nombre de fonctions COOH du polymère}}$$

Ce ratio est compris entre 0,01 et 1.

Les réticulats (III) obtenus peuvent être symbolisés comme suit :

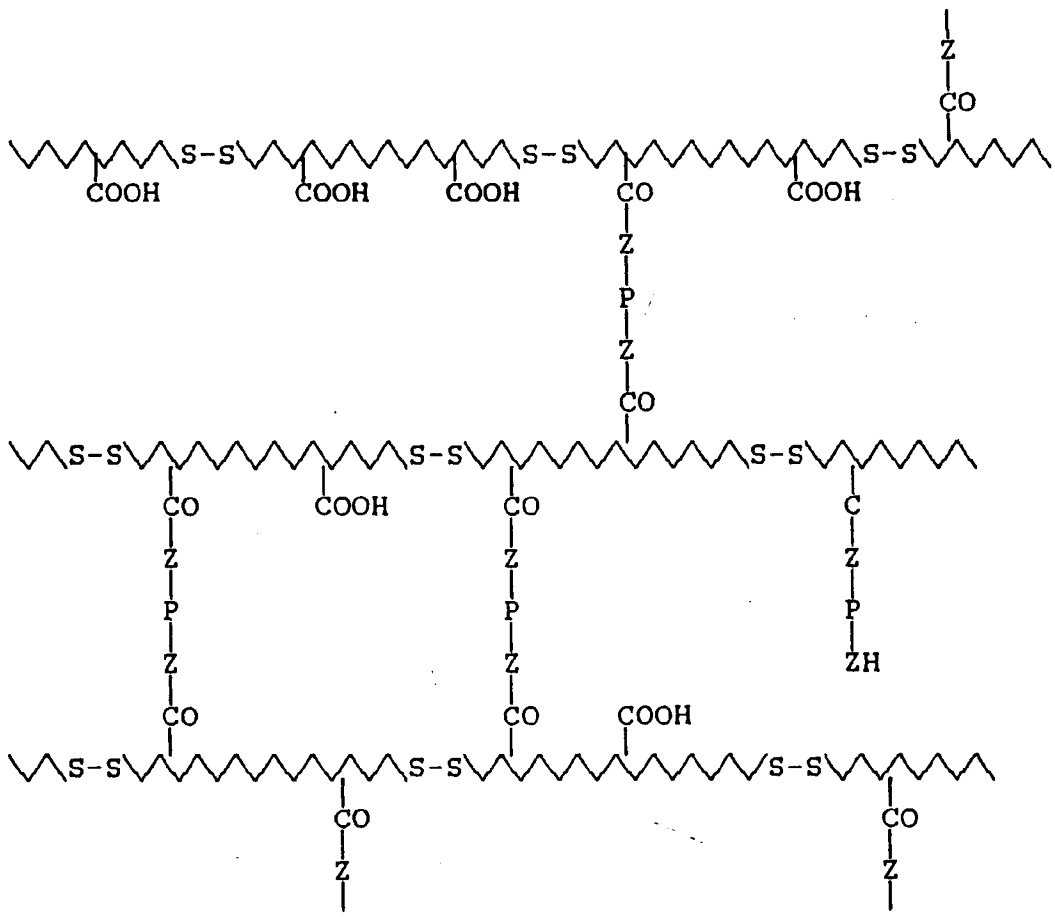

avec Z = O ou NH.

-Z-P-Z- est un pont dérivant des polyols (Z = O):HO-P-OH ou des polyamides (Z = NH):$H_2N$-P-$NH_2$.

La réduction d'un tel réticulat peut être réalisée en suspension dans l'eau en présence de dithiothréitol ou de tributylphosphine. Elle conduit à un mélange de molécules comportant plusieurs fonctions -SH qui peut être isolé, lyophilisé et conservé sous azote à une température inférieure à 0°C. Il est ensuite possible, dans des conditions d'oxydation douces, de reformer les ponts disulfures pour obtenir un réticulat semblable à (III).

Dans le cas particulier où l'agent de pontage est choisi parmi les produits suivants : cystamine ou esters de la cystine ou de l'homocystine, les groupements P du réticulat (III) comportent, également, des ponts disulfures et la réduction du réticulat conduit alors à un mélange composé majoritairement des molécules di, tri et tetra-SH décrites plus haut (formule I).

La dernière phase du procédé, commune aux deux variantes sus-évoquées, consiste à oxyder les oligomères SH obtenus à l'étape précédente, de manière à produire des polymères, dont notamment ceux de formule (II), et/ou des réticulats (III), en (re)formant des ponts disulfures.

Cette oxydation s'effectue, soit et, de préférence, en présence d'au moins un système oxydant comprenant, par exemple, l'iode et/ou ses dérivés et/ou l'eau oxygénée ou un système enzymatique, soit par électrochimie, soit directement à l'air.

La présente invention vise, également, toute composition adhésive formée par un mélange d'au moins deux produits de formule (I) et/ou (II) et/ou (III).

En particulier, les compositions intéressantes sont celles comprenant des mélanges d'oligomères (I), car une fois reoxydés ceux-ci conduisent à des biomatériaux, gels, revêtements multi-SH, décrits ci-dessus. Ces composés réoxydés doivent présenter un certain nombre de propriétés mécaniques, en relation avec leurs caractéristiques d'usage. Le niveau des propriétés mécaniques est essentiellement dépendant de la structure du réseau formé et de la maîtrise de la réticulation des multi-fonctions, de préférence multi-SH des oligomères. En théorie, toute composition de multi-SH, de fonctionnalité moyenne en SH strictement supérieure à 2, peut donner un réticulat insoluble. La fonctionnalité moyenne en SH peut être définie comme suit :

$$F_{moyenne} = \text{nombre de motif SH par molécule} = \frac{A}{B}$$

avec :

- A = 1. nombre de molécules mono-SH + 2. nombre de molécules di-SH + 3. nombre de molécules tri-SH 4. nombre de molécules tétra-SH,
- B = nombre de molécules mono-SH + nombre de molécules di-SH + nombre de molécules tri-SH + nombre de molécules tétra-SH.

Compte tenu de la possibilité de réactions intramoléculaires qui perturbent la formation du réseau en consommant des noeuds potentiels, il est préférable de viser des $F_{moyenne}$ pour les mélanges d'oligomères de l'ordre de 2,1 à 2,5, pour assurer la formation du réseau. De façon générale, l'élasticité et le gonflement (aspect gel) du réticulat diminue lorsque la $F_{moyenne}$ augmente.

Une fonctionnalité moyenne souhaitée (par exemple 2,3) peut être obtenue directement ou indirectement.

Selon la méthode directe, on réticule les polycondensats linéaires de longueur connue, afin d'estimer la proportion relative de mono-SH par rapport aux di-SH, avec une quantité adaptée d'agent pontant, tel que la cystine diméthylester. Après réduction du réticulat obtenu, on dispose d'un mélange de mono, di, tri, tetra-SH dont la $F_{moyenne}$ sera proche de celle désirée. Il faut s'assurer, toutefois, de la réactivité totale de l'agent pontant et de la totalité de la réduction des ponts SS.

La méthode indirecte consiste à "sur-réticuler" un polymère linéaire en visant une $F_{moyenne}$ théorique, voisine de 3 par exemple, à réduire ce réticulat, à déterminer par dosage la $F_{moyenne}$ obtenue, à préparer, par réduction d'un polycondensat linéaire, un mélange de mono et de di-SH proche de 2 et à obtenir, en mélangeant les deux compositions dosées dans des proportions voulues, la $F_{moyenne}$ correspondant à un optimum des propriétés recherchées.

Pour les applications de biomatériaux nécessitant la formation d'un gel, il paraît souhaitable de partir d'une composition, i. e. d'un mélange d'oligomères ayant une $F_{moyenne}$ supérieure ou égale à 2, de préférence inférieure ou égale à 2,6 et, plus préférentiellement encore, à 2,3.

Pour des biomatériaux adhésifs plus durs, il paraît souhaitable de viser des $F_{moyennes}$ supérieures ou égales à 2,3 et, préférentiellement, à 2,5.

Les oligomères (I), polymères (II) entre autres, réticulats (III), fonctionnalisés ou non, sont des composés ne présentant aucune toxicité directe ou indirecte : ils ne sont pas carcinogènes, tératogènes, immunogènes ni mutagènes. Par ailleurs, ils sont parfaitement biodégradables, c'est-à-dire qu'ils sont constitués de produits s'intégrant parfaitement bien dans les voies métaboliques (cycle de Krebs notamment) de l'être humain ou animal. Les produits de dégradation de ces composés sont ipso facto parfaitement tolérés.

En particulier, il est intéressant de noter que les oligomères (I) ont une faible masse molaire (inférieure à 1 000 Da) et qu'ils sont ainsi capables de diffuser à l'intérieur des tissus biologiques pour y être ensuite polymérisés et/ou réticulés. Les enchevêtrements qu'ils forment alors avec les glycoprotéines assurent un lien adhésif solide.

Sous forme réduite et associés à un système oxydant, ces produits et/ou leurs mélanges conviennent très bien à titre de biomatériaux adhésifs ou de colles biologiques. Ces constituants rentrent dans le champs de l'invention.

Sous forme oxydée, ces constituants sont des réseaux cohésifs, parsemés de ponts disulfures et ayant entre eux des propriétés mécaniques et biologiques modulables.

L'invention a également pour objet l'utilisation des produits de formule (I), (II), ou des réticulats (III) pour la préparation d'une composition adhésive, biocompatible, biodégradable et non toxique, à usage chirurgical.

Les exemples 1 à 20 qui suivent sont une illustration des propriétés et des variantes des adhésifs selon l'invention. Ils décrivent, également, les structures et les méthodes de préparation des produits entrant dans

la composition des adhésifs selon l'invention.

EXEMPLES

**EXEMPLE 1 :** SYNTHESE DU POLYMERE (1) PAR POLYCONDENSATION EN SOLUTION DANS LE DIMETHYLACETAMIDE (DMAC) DU CHLORHYDRATE DE LA CYSTINE DIMETHYLESTER ET DU CHLORURE DE SUCCINYLE.

(1) 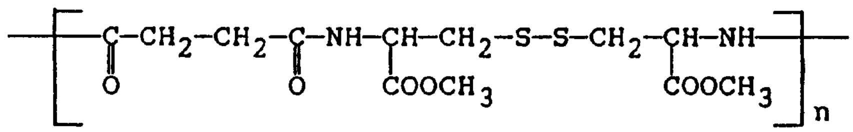

25 g (0,073 mol) de chlorhydrate de cystine diméthylester et 400 ml de DMAC sont placés dans un réacteur de 1 l. 41,2 ml de triéthylamine (0,293 mol) sont alors ajoutés. 8,1 ml de chlorure de succinyle fraichement distillé sont dilués dans 100 ml de DMAC, le tout est ajouté au mélange réactionnel à l'aide d'une ampoule de coulée. Le mélange réactionnel est alors agité pendant 24 heures à température ambiante. Le sel de triéthylammonium précipité est enlevé par filtration, puis le mélange réactionnel est précipité dans 5 l d'eau. Le polymère est récupéré par filtration, séché à l'étuve sous vide : 13 g d'une poudre blanche (légèrement rosée) sont ainsi obtenus. Les spectres RMN $^1H$ (dans l'acide trifluoroacétique deutéré (TFA)) et IR sont conformes. Les masses molaires, déterminées par chromatographie d'exclusion stérique (SEC) dans le DMAC et exprimées en équivalents polystyrène, sont les suivantes :

$M_a$ = 6 200, $M_w$ = 9 600

**EXEMPLE 2 :** SYNTHESE DU POLYMERE (1) PAR POLYCONDENSATION INTERFACIALE EAU/TOLUENE DU CHLORHYDRATE DE LA CYSTINE DIMETHYLESTER ET DU CHLORURE DE SUCCINYLE.

25 g (0,073 mol) de chlorhydrate de cystine diméthylester et 200 ml de DMAC sont placés dans un réacteur de 1 l. 31,06 g de carbonate de sodium anhydre (0,293 mol) sont alors ajoutés. Une préémulsion est ensuite formée par addition de 100 ml de toluène. 8,1 ml de chlorure de succinyle fraîchement distillé sont alors dilués dans 100 ml de toluène, le tout est ajouté au mélange réactionnel à l'aide d'une ampoule de coulée. Le mélange réactionnel est alors agité pendant 4 heures à température ambiante. Le polymère, précipité au cours de la réaction, est récupéré par filtration, lavé avec de l'acétone, puis avec de l'eau. Il est séché à l'étuve sous vide : 14 g d'une poudre blanche (légèrement rosée) sont ainsi obtenus. Les spectres RMN $^1H$ (dans le TFA) et IR sont conformes et analogues à ceux obtenus pour le polymère de l'exemple 1. Les masses molaires, déterminées par SEC dans le DMAC et exprimées en équivalents polystyrène, sont les suivantes :

$M_a$ = 5 700, $M_w$ = 11 500

**EXEMPLE 3 :** HYDROLYSE DES FONCTIONS ESTER DU POLYMERE (1) : OBTENTION DU POLYMERE (2)

(2) 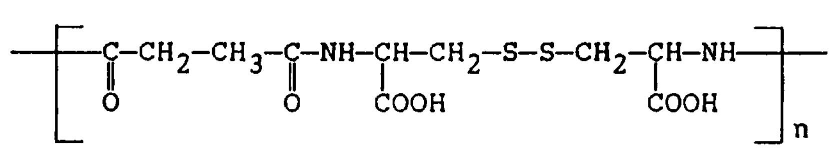

5 g de polymère (1) obtenu par polycondensation en solution ou interfaciale sont mis en suspension dans 1 l d'eau. Le pH est ajusté à 10,5 avec de la soude 1 M et maintenu à cette valeur pendant toute la durée de l'hydrolyse. L'addition de soude est arrêtée lorsque la solution est devenue limpide. La solution est alors acidifiée jusqu'à un pH < 3 par une résine échangeuse d'ions acide. Elle est concentrée, congelée, puis lyophilisée. 4,6 g d'une poudre blanche sont obtenus. Les spectres RMN $^1H$ (dans le TFA et dans $D_2O$) et IR sont conformes et montrent que l'hydrolyse des fonctions esters est totale.

**EXEMPLE 4 :** REDUCTION DU POLYMERE (2) PAR LE DITHIOTHREITOL : OBTENTION DE LA MOLECULE (3)

(3) 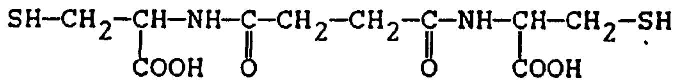

3 g de polymère (2) et 2,87 g de dithiothréitol (DTT) sont dissous dans 70 ml d'eau sous atmosphère d'azote. Le pH est ajusté à 8,5 par addition de soude 1 M et la solution est agitée pendant 3 heures sous bulle à bulle d'azote. Le mélange est alors extrait deux fois par 100 ml d'acétate d'éthyle. La phase aqueuse est ensuite acidifiée par une résine échangeuse d'ions acide jusqu'à pH = 4,5, puis concentrée et précipitée dans un excès d'acétone. Le précipité collant obtenu est redissous dans un minimum d'eau et reprécipité dans l'acétone. Il est finalement redissous dans l'eau et lyophilisé. 2 g d'un produit légèrement jaune sont récupérés. Le spectre RMN $^1H$ (dans $D_2O$) obtenu est conforme à la formule (3), les groupements carboxyliques étant sous forme ionisée.

**EXEMPLE 5 :** REDUCTION DU POLYMERE (2) PAR LA TRI(N-BUTYL)PHOSPHINE : OBTENTION DE LA MOLECULE (3)

2,4 g de polymère (2) sont dissous dans 30 ml d'eau sous atmosphère d'azote. 120 ml de méthanol, préalablement dégazé, sont alors ajoutés. Puis, 2 ml de tri(n-butyl) phosphine sont injectés dans le mélange réactionnel. Après 3 heures de réaction, le méthanol est évaporé à l'aide d'un évaporateur rotatif. 50 ml d'eau sont rajoutés à la solution aqueuse résiduelle qui est ensuite extraite deux fois avec 200 ml d'acétate d'éthyle. La solution aqueuse est ensuite acidifiée et précipitée dans l'acétone, comme décrit dans l'exemple 4. Le spectre RMN $^1H$ dans $D_2O$ est identique à celui du produit obtenu dans l'exemple 4.

**EXEMPLE 6 :** RETICULATION DU POLYMERE (2) PAR LA CYSTINE DIMETHYLESTER

5 g du polymère (2) et 5,3 g de chlorhydrate de cystine diméthylester sont dissous dans 100 ml d'eau. 6 g de N-diméthylaminopropyl, N'-éthyl carbodiimide (EDC) sont alors dissous dans 5 ml d'eau et aussitôt rajoutés dans le mélange réactionnel. Le mélange prend immédiatement une coloration rouge foncé puis, au bout de quelques secondes, il se forme un précipité rose. La réaction est arrêtée après 3 heures et 200 ml d'eau sont rajoutés. Le précipité est récupéré par filtration, lavé plusieurs fois avec de l'eau, puis séché à l'étuve sous vide.

**EXEMPLE 7 :** RETICULATION DU POLYMERE (2) PAR LA CYSTINE DIETHYLESTER

5 g du polymère (2) et 5,73 g de chlorhydrate de cystine diéthylester sont dissous dans 100 ml d'eau. 6 g de N-diméthylaminopropyl, N'-éthyl carbodiimide (EDC) sont alors dissous dans 5 ml d'eau et aussitôt rajoutés dans le mélange réactionnel. La réaction est arrêtée après 3 heures et 200 ml d'eau sont rajoutés. Le précipité est récupéré par filtration, lavé plusieurs fois avec de l'eau puis séché à l'étuve sous vide.

**EXEMPLE 8 :** REDUCTION PAR LE DITHIOTHREITOL DU POLYMERE RETICULE DE L'EXEMPLE 6

1 g du polymère réticulé de l'exemple 7 et 1,1 g de dithiothréitol sont dissous dans 50 ml d'eau, préalablement purgée par un courant d'azote. Le pH est ajusté à 9,5 par la soude 1 M. Le mélange réactionnel devient limpide et la réaction est arrêtée au bout d'une heure. Après six extractions avec 50 ml d'acétate d'éthyle, la solution aqueuse est acidifiée jusqu'à pH = 5 par une résine échangeuse, réextraite avec deux fois 50 ml d'acétate d'éthyle puis lyophilisée. Le produit obtenu est un mélange comprenant, principalement, les molécules (3), (4), (5) suivantes :

(3) 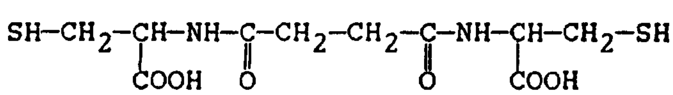

(4)

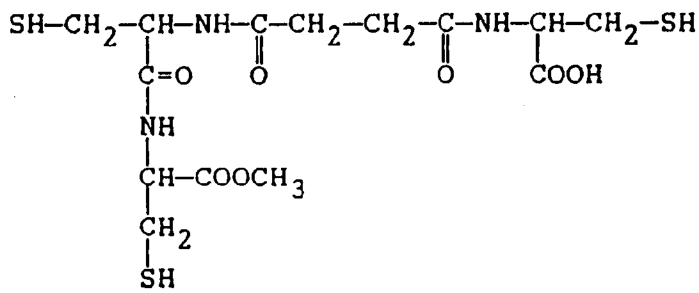

(5)

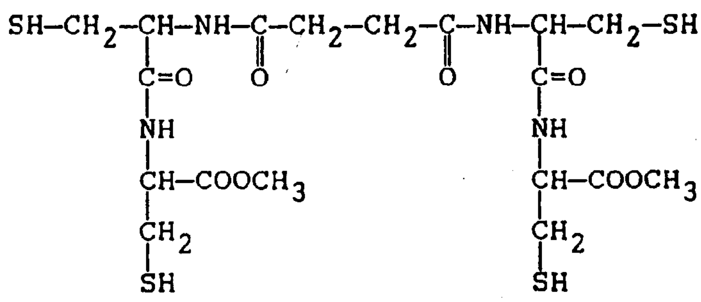

Les fonctions carboxyliques sont sous forme ionisée (-$COO^-$, $Na^+$). Le mélange n'est plus entièrement soluble dans l'eau lorsque le pH est < 3.

**EXEMPLE 9** : REDUCTION PAR LE DITHIOTHREITOL DU POLYMERE RETICULE DE L'EXEMPLE 6 - HYDROLYSE DES FONCTIONS ESTER DU PRODUIT OBTENU

La réaction est réalisée, comme décrit à l'exemple 8, mais la solution réduite est maintenue à pH = 9,5 pendant 24 heures à 35° C. Après six extractions avec 50 ml d'acétate d'éthyle, la solution aqueuse est acidifiée jusqu'à pH = 5 par une résine échangeuse, réextraite avec deux fois 50 ml d'acétate d'éthyle, puis lyophilisée. Le produit obtenu est un mélange comprenant, principalement, les molécules (3), (6), (7) suivantes :

(3)

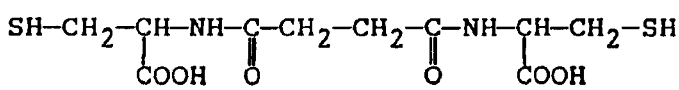

(6)

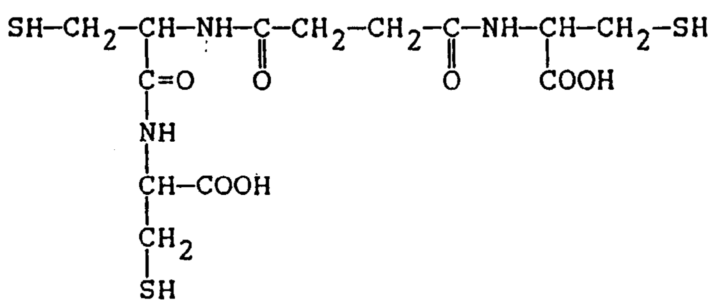

(7) 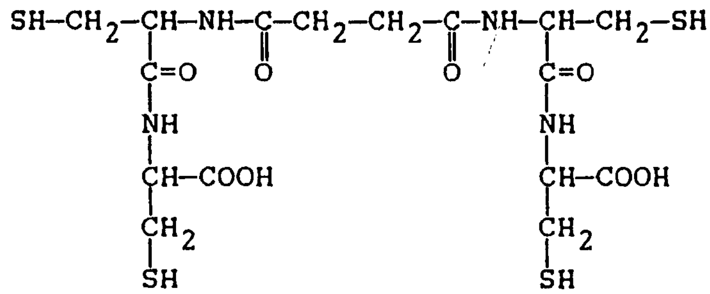

Les fonctions carboxyliques sont sous forme ionisée (-$COO^-$, $Na^+$). Le mélange peut être acidifié (jusqu'à pH = 2,5) par passage sur une résine échangeuse d'ions. Dans ce cas, la solubilité dans l'eau est conservée.

**EXEMPLE 10 :** REDUCTION PAR LE DITHIOTHREITOL DU POLYMERE RETICULE DE L'EXEMPLE 7

1 g du polymère réticulé de l'exemple 7 et 1,1 g de dithiothréitol sont dissous dans 50 ml d'eau, préalablement purgée par un courant d'azote. Le pH est ajusté à 9,5 par de la soude 1 M. Le mélange réactionnel devient limpide et la réaction est arrêtée au bout d'une heure. Après six extractions avec 50 ml d'acétate d'éthyle, la solution aqueuse est acidifiée jusqu'à pH = 4 par une solution d'HCl 1N. Un précipité collant, légèrement marron est obtenu. Il s'agit d'un mélange constitué, principalement, des molécules (3), (8), (9) suivantes :

(3) 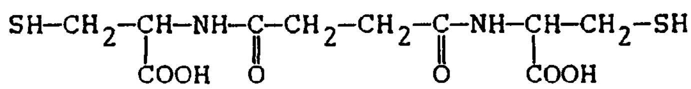

(8) 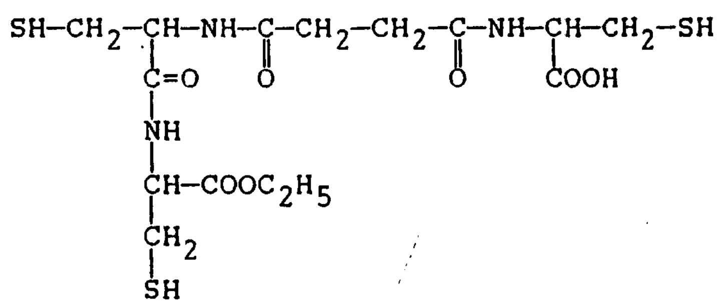

(9) 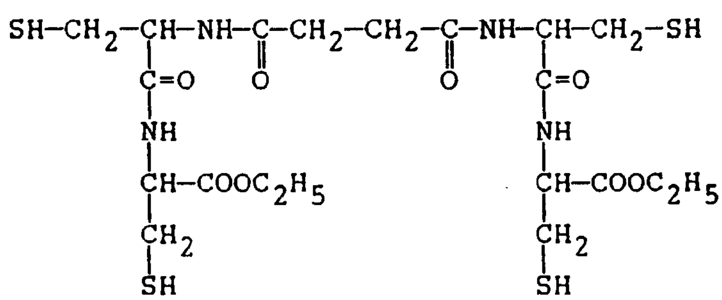

**EXEMPLE 11 :** EVALUATION EX VIVO DE L'ADHESION TISSULAIRE.

L'évaluation des propriétés adhésives de composition selon l'invention a été réalisée sur des tissus musculaires de lapin (râble). Ces tissus sont conservés à 4°C dans du sérum physiologique pendant 48 heures au maximum. Le tissu de lapin est découpé dans le sens des fibres à l'aide d'une trancheuse électrique (épaisseur des tranches : 2,5 ± 0,5 mm), puis des carrés de 25 mm x 25 mm sont découpés dans les tranches obtenues.

Les essais sont réalisés sur un appareil de traction usuel, par exemple de type Adamel Lhomargy de type DY34 muni d'un capteur de force de 100 N. Cet appareil permet d'obtenir les courbes force-déplacement. Il permet également d'obtenir la force maximale d'arrachement ($F_{max}$), le module de Young, et l'énergie mise en jeu peut être calculée d'après l'aire sous la courbe.

Dans chaque type d'essai, deux éprouvettes de tissu de lapin sont fixées à l'aide d'une colle cyanoacrylique (par exemple vendue sous la marque Loctite superglue, liquide ou gel) sur des supports inertes, verres ou cartons très rigides de dimension supérieure. Les tests sont réalisés au bout de 3 minutes après une pression de 4 N.

La composition utilisée est une solution du polymère, à raison de 100 µl par échantillon, (2) selon l'exemple 3, à une concentration de 20 à 25 pour cent et, en variante, non pas en solution mais directement en poudre sur le tissu de lapin. Après 3 minutes de collage sous pression, on obtient une valeur d'adhésion ($F_{max}$) comprise entre 1,5 et 2 N. Cette valeur est intéressante et peut être comparée aux valeurs équivalentes de 1,5 N à 2,5 N des colles biologiques classiques à base de fibrine. Le même test effectué avec des polymères carboxyliques commerciaux tels que polyacide acrylique, alginate de sodium, montre un faible niveau d'adhésion ($F_{max}$ < 1 N, travail d'adhésion < 1 J).

**EXEMPLE 12 :** COMPOSES MULTI-SH.

Les tests sont effectués sur des composés multi-SH sous forme -COOH, à savoir les composés (3), (4) et (5), (6), (7), (8), (9) des exemples 4, 5, 8, 9 et 10, de préférence (3), (6) et (7).

Pour cela les tissus d'éprouvette sont préalablement imprégnés par une solution hydroalcoolique d'iode puis la composition est ajoutée en solution dans l'eau à raison de 100 µl par échantillon. Selon les éprouvettes et les essais les forces d'arrachement mesurées sont comprises entre 1,12 et 3,46N, la moyenne étant de 2,04 ± 0,62 N.

Ces résultats sont donc comparables et même en moyenne supérieurs à ceux des colles classiques à base de fibrine. En outre l'aire sous la courbe, c'est-à-dire l'énergie mise en oeuvre, est plus importante ou égale à celle des meilleures colles de fibrine.

Les essais réalisés après une heure de contact montrent que la force d'adhésion augmente avec le temps (entre 3 et 4 N au bout d'une heure).

**EXEMPLE 13**

0,1 gramme de collagène humain de type IV (IMEDEX), mis en solution dans 4 ml d'eau, est réduit à pH 9 par 10 mg de dithiothréitol (DTT) sous atmosphère inerte pendant 18 heures. Après dialyse de la solution (3 fois 8 heures), on ajoute 80 mg de dérivés de type "multi-SH" préparés à l'exemple 9. Le pH de la solution est ajusté à 9 par ajout de soude concentrée, 20 microlitres d'eau oxygénée à 35 % en poids sont ajoutés à la solution pour permettre une oxydation partielle du mélange. Après 30 minutes, le mélange réactionnel est testé en adhésion par application sur tissus de lapin suivant le protocole décrit à l'exemple 12.

On enregistre après 3 minutes de contact sous 4 N :

- une force adhésive moyenne de 1,9 +/- 0,4 N et
- une énergie d'adhésion de 2,9 +/- 1,2 mJ.

Les exemples suivants ont trait à des procédés de liaison de tissus entre eux ou de liaison d'un tissu et d'un biomatériau implanté par superposition de deux surfaces et dans lequel on applique ou laisse diffuser sur au moins l'une des surfaces une composition selon l'invention et/ou un agent oxydant dans des conditions telles que l'agent oxydant entraîne une polymérisation de la composition lorsque les surfaces sont appliquées l'une contre l'autre. Cette application peut être effectuée par exemple par pulvérisation ou imprégnation de la composition selon l'invention ou bien on peut interposer entre les deux surfaces un biomatériau résorbable selon l'invention contenant un oxydant, les deux surfacés ayant été imprégnées de la composition selon l'invention ou bien on peut interposer une composition sous forme de biomatériau selon l'invention entre les deux surfaces à réunir.

**EXEMPLE 14**

On dépose 150 microlitres d'une solution de monomère adhésif (3), (4), (5), (6), (7), (8) ou (9), (60 mg) selon l'un des exemples 4, 5, 8, 9 et 10, de préférence 9. On place une compresse contenant une solution oxydante, par exemple une solution d'iode ou une solution d'eau oxygénée, entre les deux tissus et on réunit

les deux tissus de part et d'autre de la compresse en collagène en maintenant une pression pendant une durée de préférence de quelques minutes. Au lieu d'être en collagène, la compresse peut être, par exemple, en alginate, en acide hyaluronique ou en cellulose oxydée.

**EXEMPLE 15 :** ADHESION DE LA PEAU EN CHIRURGIE PLASTIQUE ET REPARATRICE.

On badigeonne le plan musculaire à l'aide de la solution oxydante puis, après avoir élimi-né le surplus de solution à l'aide d'une compresse, on pulvérise une composition adhésive liquide selon l'invention sur le plan musculaire après quoi on réunit les deux tissus.

Cet exemple est applicable à toute chirurgie pour le maintien de deux tissus de manière cohésive, une ou les deux surfaces pouvant être badigeonnées, soit de la solution oxydante, soit de la composition selon l'invention, après quoi on effectue une pulvérisation, soit de la composition, soit de la solution oxydante, avant de réunir les tissus.

**EXEMPLE 16 :** PROTECTION D'ANASTOMOSES.

L'invention peut être mise en oeuvre pour la protection d'anastomoses lors de chirurgie vasculaire, viscérale, gynécologique ou urologique. Après pulvérisation d'une solution de composition de monomère adhésif selon l'invention à la surface de l'anastomose, on place sur le tissu recouvert ainsi de monomère adhésif une compresse ou un patch de compression d'anastomose imprégné de la solution oxydante.

En variante on peut entourer l'anastomose d'une compresse ou patch d'anastomose préimprégné de monomère adhésif puis on pulvérise la solution oxydante sur le matériau pour obtenir son adhésion au tissu.

**EXEMPLE 17 :** COMBLEMENT TISSULAIRE.

Suite à une exérèse de tissu mou ou d'os, la cavité à combler est recouverte de monomère adhésif selon l'exemple 9 puis une suspension de microbilles de collagène partiellement imprégnée de la solution oxydante est mise en place pour combler la cavité.

En variante on peut combler la cavité par une suspension de microbilles ou une solution de collagène imprégnée du monomère adhésif puis on injecte, au sein du volume comblé, une solution oxydante aqueuse d'iode.

**EXEMPLE 18 :** RECONSTITUTION TISSULAIRE.

Lors de la chirurgie de la dure-mère, après exérèse du tissu pathologique, on met en place un patch, par exemple en collagène, pour remplacer le tissu excisé et autoriser la formation d'un nouveau tissu. On effectue un dépôt de composition de monomère adhésif sur le pourtour du patch puis on le met en place et l'on pulvérise la solution oxydante sur le patch pour assurer l'adhésion et l'étanchéité du collage.

**EXEMPLE 19 :** PROTECTION DE TISSU.

Pour protéger un tissu interne tel qu'une muqueuse ou un tissu externe, ou pour améliorer une cicatrisation, on imprègne le tissu de la préparation d'oxydant, on élimine, s'il y a lieu, le surplus de solution d'oxydant et on pulvérise une composition de monomère adhésif. Eventuellement on peut ensuite mettre en place une compresse protectrice, par exemple en collagène.

**EXEMPLE 20 :** PROCEDE D'HEMOSTASE.

En cas de saignement important en chirurgie cardiovasculaire, abdominale ou thoracique, par exemple lors d'hépatectomie, on pulvérise une solution de monomère adhésif selon l'invention sur la tranche de tissu et on applique une compresse préimprégnée de solution oxydante et l'on maintient la compression jusqu'à l'obtention de l'hémostase.

Le cas échéant, notamment en cas de saignement peu important, l'hémostase peut être obtenue par la seule application par pulvérisation de monomère adhésif et de la solution oxydante de manière successive ou concomittante.

## Revendications

1. Composition adhésive, biocompatible, biodégradable et non toxique, à usage chirurgical, notamment pour la liaison de tissus biologiques entre eux ou d'un tissu et d'un biomatériau implanté, caractérisée en ce qu'elle comporte un produit organique contenant au moins deux fonctions thiols ou dérivés et des fonctions carboxyliques, protégées ou non, et/ou des fonctions carbonylées, de formule générale suivante :

(I) 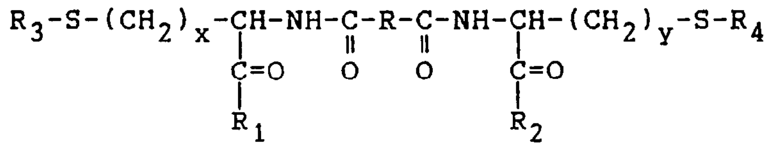

dans laquelle : x, y, z = 1 ou 2 et $\phi$ est un cycle aromatique,

- R est une chaîne hydrocarbonée, de préférence alkylée, comportant de 1 à 50 atomes de carbone et, plus préférentiellement encore, aliphatique ayant de 1 à 10 atomes de carbone,
- $R_1$ et $R_2$ sont identiques ou différents et sont choisis parmi les groupements suivants :
  $-O-R_5$ ;

  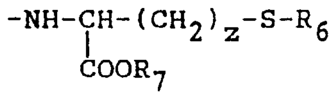 ;

  $-NH-(CH_2)_y-S-R_6$
- $R_3$, $R_4$, $R_6$, $R_6$, et $R_7$, représentent, indépendamment, l'hydrogène ou un groupe aliphatique et/ou alicyclique et/ou aromatique, de préférence un groupe alkyle inférieur et/ou un groupe aromatique et, plus préférentiellement encore, l'un des groupements suivants :
  $-CH_3$ ; $-CH_2-CH_3$ ; $-CH_2-\phi$ ;

  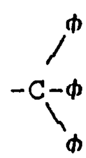

2. Composition selon la revendication 1, caractérisée en ce que $R_1$ et $R_2$ représentent $- O - R_6$.

3. Composition selon la revendication 1, caractérisée en ce que $R_1$ représente :

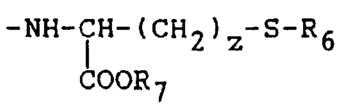

et $R_2$ représente $-O-R_5$ ou inversement.

4. Composition selon la revendication 1, caractérisée en ce que $R_1$ et $R_2$ sont constitués par le radical :

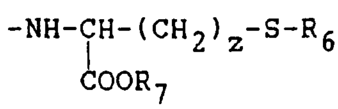

5. Composition selon l'une quelconque des revendications 1 à 4, caractérisée en ce que R est choisi de telle sorte que le radical :

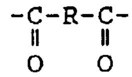

de la formule (I) soit un radical appartenant à la famille des restes d'acides poly, avantageusement dicarboxyliques, à l'exclusion de l'acide citrique, R étant de préférence sélectionné parmi les groupements suivants :

$-(CH_2)_p$ ;

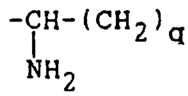 ; 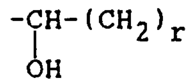

avec :
- p ≦ 5, de préférence, égal à 2 ou à 3,
- q ≦ 5, de préférence, égal à 1 ou à 2,
- et enfin, r ≦ 5, de préférence, égal à 1.

**6.** Composition selon l'une quelconque des revendications 1 à 4, caractérisée en ce que R est composé d'enchainement polylactique et/ou polyglycolique et/ou polyaminoacide, de faible masse moléculaire.

**7.** Composition selon l'une des revendications 1 à 6, caractérisée en ce qu'elle se présente sous forme de solution liquide, de spray, de gel, de particules ou d'un matériau massif, biorésorbable.

**8.** Biomatériau sous forme de prothèse ou non, caractérisé en ce qu'il présente, superficiellement ou dans sa profondeur, une composition selon l'une des revendications 1 à 7, permettant d'assurer l'adhésion dudit biomatériau à un tissu biologique.

**9.** Biomatériau sous forme de prothèse ou non, caractérisé en ce qu'il présente, superficiellement ou dans sa profondeur, un agent oxydant destiné à réagir avec la composition selon l'une des revendications 1 à 7, pour assurer l'adhésion dudit biomatériau à un tissu biologique, ledit agent oxydant comprenant, de préférence de l'iode et/ou ses dérivés et/ou de l'eau oxygénée.

**10.** Biomatériau selon l'une des revendications 8 et 9, caractérisé en ce qu'il se présente sous forme de patch, compresse, matériau de comblement, ciment chirurgical ou prothèse, notamment vasculaire, osseuse, tissulaire ou ligamentaire, ou implant.

**11.** Biomatériau selon l'une quelconque des revendications 8 à 10, caractérisé en ce qu'il est constitué, au moins en partie, par un polymère qui répond à la formule générale suivante :

(II) 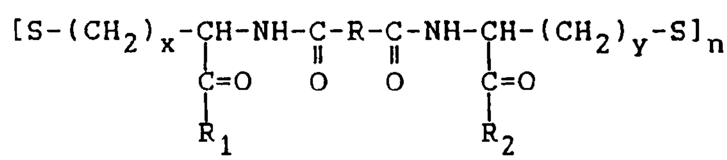

n est compris entre 1 et 100 et dans laquelle $R_1$ et $R_2$ sont tels que définis ci-dessus selon l'une quelconque des revendications 1 à 4 et R est tel que défini selon l'une quelconque des revendications 5 et 6.

**12.** Biomatériau selon l'une quelconque des revendications 8 à 10, caractérisé en ce qu'il est constitué, au moins en partie, par un réticulat constitué par un ou des polymères définis dans la revendication 8, réticulés par des ponts issus d'au moins un agent de pontage, de préférence choisi parmi les polyols et/ou les polyamines et, plus préférentiellement encore, parmi les produits suivants cystine et ses dérivés, oses et leurs dérivés hydrogénés, autre polyols (glycérol).

**13.** Biomatériau caractérisé en ce qu'il est constitué par un mélange d'au moins deux des produits définis dans l'une quelconque des revendications 1 à 12 pour assurer l'adhésion dudit biomatériau à un tissu biologique.

**14.** Biomatériau selon la revendication 13, caractérisé en ce que les monomères constitutifs contiennent un nombre de SH moyen supérieur ou égale à 2.

**15.** Matériau adhésif selon l'une quelconque des revendications 1 à 8, 10 à 14, caractérisé par une association d'au moins l'un des produits et/ou polymères et/ou réticulats, et/ou compositions selon au moins l'une des revendications 1 à 7 avec des macromolécules biologiques, ou des polymères synthétiques ou naturels biodégradables, tels que :

- les polysaccharides ; e.g. amidon, cellulose, chitosane, dextrane, mucopolysaccharides tels que l'acide hyaluronique ou chondroïtine sulfate ;
- les protéines ; e.g. collagène, gélatine, albumine, globulines ;
- les polyaminoacides ;
- les polyesters (notamment lactiques et/ou glycoliques), les polyorthoesters, les polyanhydrides, les polyphosphazènes ;
- et les lipides et phospholipides.

Office européen des brevets

# RAPPORT DE RECHERCHE EUROPEENNE

Numero de la demande
EP 94 40 1631

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 049 469 (DR. RUHLAND NACHF. GMBH)<br>* revendications; exemples *<br>--- | 1 | A61L25/00<br>A61L27/00<br>A61L31/00 |
| D,A | EP-A-0 332 530 (SANOFI)<br>* revendications *<br>----- | 1 | |
| | | | **DOMAINES TECHNIQUES RECHERCHES (Int.Cl.6)** |
| | | | A61L |

Le présent rapport a été établi pour toutes les revendications

| Lieu de la recherche | Date d'achèvement de la recherche | Examinateur |
|---|---|---|
| LA HAYE | 6 Septembre 1994 | ESPINOSA, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)